# EUROPÄISCHE PATENTANMELDUNG

(11) **EP 4 173 676 A1**
(43) Veröffentlichungstag der Anmeldung: **03.05.2023**
(21) Anmeldenummer: 21204968.8
(22) Anmeldetag: 27.10.2021
(51) Int. Cl.: A61Q 19/08, A61Q 19/00, A61Q 17/04, A61K 8/9789

(54) **KOSMETISCHE ZUSAMMENSETZUNG ENTHALTEND EINEN EXTRAKT AUS GENTIANA UND LEONTOPODIUM UND / ODER CENTELLA**

(71) Anmelder: Weleda AG, 4144 Arlesheim (CH)
(72) Erfinder: BACH, Petra, 70329 Stuttgart (DE); BÄSSLER, Dagmar, 73529 Schwäbisch Gmünd (DE); HÄNNI-CIUNEL, Katarzyna, 4312 Magden (CH); IDOUX, Alicia, 4112 Bättwil (CH); IRRGANG, Bernhard, 5737 Menziken (CH)
(74) Vertreter: Simandi, Claus

(57) **Zusammenfassung**

Die Erfindung betrifft eine kosmetische Zusammensetzung enthaltend Gentiana (Enzian) und Leontopodium (Edelweiss) und / oder Centella (indischer Wassernabel), und dessen Verwendung, insbesondere als Anti-Ageing /Anti Wrinkle Mittel.

Gentiana, Leontopodium und Centella sind geeignet, die Hautalterung zu verzögern, indem sie überraschenderweise die Protease MMP-1, die u.a. verantwortlich ist für den Abbau von Kollagen in der Dermis, hemmen sowie die Kollagen-1 Expression im Bindegewebe anregen können.

## Beschreibung

Die Erfindung betrifft eine kosmetische Zusammensetzung enthaltend Gentiana, Leontopodium und / oder Centella, und dessen Verwendung, insbesondere als Anti-Ageing Mittel.

Weleda AG forscht mit modernen und in Verbindung mit anthroposophischen Methoden an vorteilhaften natürlichen Pflanzenstoffen für die Kosmetik.

Die Pflanzengattung Gentiana (Enzian) aus der Familie der Enziangewächse (Gentianaceae) mit ca. 400 Arten weist in den oberirdischen Teilen der Pflanze oder in der Wurzel charakteristische und in signifikanten Mengen vorliegende Bitterstoffe aus der Pflanzenstoffgruppe sowohl Iridoide, insbesondere Secoiridoidglykosiden, wie Swertiamarin, Amarogentin und Gentiopikrosid, als auch Flavonoide, insbesondere iso-Orientin, iso-Vitexin, iso-Orientin-4'-O-Glucosid, iso-Saponarin, auf. Das Vorkommen liegt vorrangig in den Hochgebirgen. Bekannt sind vor allem gelber Enzian (*Gentiana lutea*) und diverse Spezien vom "blauen Enzian" mit charakteristischen blauen Kelchblüten der Arten *Gentiana alpina, Gentiana acaulis, Gentiana clusii, Gentiana brachyphylla, Gentianan orbicularis* und *Gentiana septemfida var.*

Die Gattung Leontopodium (Edelweiss) kommt ebenfalls im Hochgebirge vor und weist in den oberirdischen Teilen der Pflanze charakteristisch Leontopodiumsäuren (Edelweißsäuren) und Chlorogensäure, Lignane, insbesondere Leoligin und 5-Methoxyleoligin, Flavonoide, wie Luteolin-4'-O-glucosid, Apigenin-7-O-glucosid und Luteolin und einen hohen Gehalt an Tanninen auf. Bekannt sind vor allem die Arten *Leontopodium alpinum (sog. Alpenedelweiss), Leontopodium nivale* und *Leontopodium palibinianum.*

Centella (indischer Wassernabel, auch: Asiatischer Wassernabel, Tigergras oder Gotu Kola) ist eine Pflanzengattung, die in den Tropen und Subtropen heimisch ist. Charakteristische Inhaltsstoffe in den oberirdischen Teilen der Pflanze sind Triterpensaponine, insbesondere Asiaticoside und Madecasoside, Asiatsäure, Madecassische Säure, und Flavonolglykoside. Die bekannteste Art ist *Centella asiatica.*

Die Haut hat als das größte Organ des Menschen zahlreiche lebenswichtige Funktionen und bildet die Grenzfläche zur Umwelt. Sie schützt den menschlichen Organismus vor physikalischer und mechanischer Belastung (Hitze, Kälte, UV-Strahlen, Stöße, Scherkräfte) sowie davor, dass Allergene, Chemikalien und Mikroorganismen in den Körper eindringen (Barriere von außen nach innen). Andererseits verringert sie den Wasserverlust durch die Haut (Barriere von innen nach außen). Hauptfunktionen sind demnach die Barrierefunktion vor schädigenden äußeren Einflüssen sowie die Regulation des kutanen Wasserhaushaltes.

Die Barriere ist ähnlich einer Ziegelsteinmauer aufgebaut. Ihre Hornzellen bilden die "Ziegel" des Mauerwerks, der dazwischenliegende "Kitt" ist ein komplex zusammengesetzter Hydrolipidfilm, der u.a. aus Lipiden wie beispielsweise Ceramiden, aber auch aus Proteinen und Enzymen besteht. Das Zusammenspiel dieser Einzelkomponenten kann sehr leicht gestört werden, wodurch die Barrierefunktion beeinflusst wird. Ein Faktor ist hierbei das Alter - im Alter lassen u.a. die Aktivitäten von Talg- und Schweißdrüsen und die Fähigkeit der Zellregenration, nach.

Die Hautalterung per se ist ein komplexer Vorgang, der durch verschiedene Faktoren, die zudem in Wechselwirkung zueinanderstehen, beeinflusst wird und sich in den Hautschichten auf unterschiedliche Art und Weise auswirken.

Die intrinsische Hautalterung bezeichnet die Vorgänge, die sich in allen Zellen des Körpers abspielen und als biologisches Altern bezeichnet wird. Die extrinsischen Faktoren sind die Einflüsse von außen auf die Haut. Ursächlich spielt hier die Umwelt des Menschen eine Rolle. Neben ungesundem Stress, Rauchen, falscher Ernährungsweise oder Alkoholgenuss hat die UV-Strahlung den größten negativen Einfluss auf die Haut.

In der Epidermis vollzieht sich die Hautalterung in Form eines verlangsamten Zellstoffwechsels und niedrigerer Lipidproduktion. Die Folgen: die Barrierefunktion wird verschlechtert - dadurch trocknet sie aus und wird rauer - Fältchen und Falten entstehen, vor allem im Gesicht.

Die Dermis besteht hauptsächlich aus Bindegewebszellen, den sogenannten Fibroblasten, und Bindegewebsfasern aus Kollagen und Elastin. In der jungen Haut arbeiten beide Fasertypen perfekt zusammen: Das Kollagen macht das Gewebe stabil und zugfest, das gummiartige Elastin hingegen sorgt dafür, dass die Dehnbarkeit bleibt. In der Dermis baut die Haut permanent Kollagen auf und ab. Mit zunehmendem Altern überwiegen die Abbauprozesse, d.h. die Haut verliert Kollagen und Elastin. Auch bilden die Bindegewebszellen der Dermis mit der Zeit immer weniger Kollagen. Dadurch entstehen vor allem im Gesicht schneller Fältchen und Falten, die Haut wird dünner.

Die innere Hautschicht (Subkutis) besteht vor allem aus Fettzellen und speziellen Kollagenfasern, die als Verbindungselemente dienen. Diese Hautschicht ist verantwortlich, Energie zu speichern, den Körper zu polstern und zu isolieren. Auch hier finden durch die Hautalterung Prozesse statt, die Anzahl und Größe der Lipidzellen geht zurück, was ebenfalls zu dünnerer Haut und Volumenverlust führt.

Der Prozess der Hautalterung per se kann nicht aufgehalten werden, aber der Prozess der extrinsischen Alterung kann verlangsamt werden. Neben einer gesunden Lebensweise sowie entsprechender Pflege der Haut mit geeigneten Pflegeprodukten kann die Hautalterung so zumindest teilweise hinausgezögert werden.

Kollagen hat als direkter Wirkstoff in Kosmetika gute feuchtigkeitsspendende Eigenschaften. Insbesondere Kollagen Typ I ist ein fibrilläres Kollagen, der häufigste Kollagentyp, und wichtig für die Festigkeit des Bindegewebes der Haut.

Allerdings sind Kollagenmoleküle zur Applikation viel zu groß, um die Haut mit einer intakten Barriere tatsächlich zu penetrieren. Wünschenswert für die Kosmetik sind daher Wirkstoffe, die die Kollagensynthese in der Haut anregen können und/oder den Abbau des körpereigenen Kollagens hemmen können.

Im Stand der Technik sind jeweils Extrakte von Gentiana, Leontopodium oder Centella für eine Kosmetik beschrieben.

CN 111631961 A offenbart eine Feuchtigkeitsmaske für das Gesicht, welche u.a. einen Enzianwurzelextrakt (gentian root 0.2-0.5 w/w) zusammen mit alpinem Edelweiss (0.5-2 w/w) und Centella asiatica (0.5-1.5 w/w) aufweisen kann.

Überraschenderweise konnten die Erfinder*innen feststellen, dass Extrakte von i.) Gentiana und Leontopodium, oder ii.) Gentiana und Centella oder iii.) Gentiana und Leontopodium und Centella Einfluss auf die Menge an Kollagen Typ 1 in der extrazellulären Matrix haben und starke synergistische Effekte zeigen.

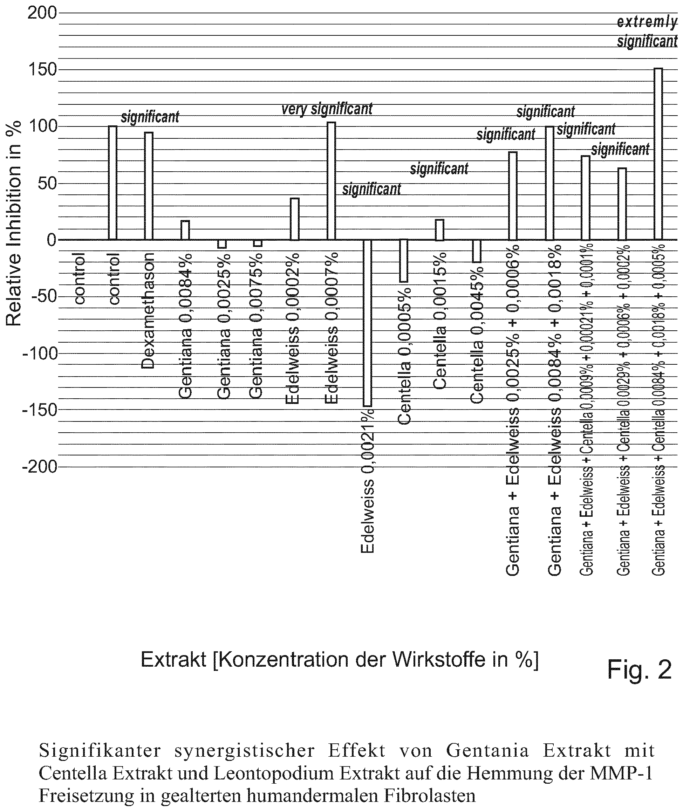

In zwei verschiedenen in-vitro Testmodellen (Assays) wurden entsprechende synergistische signifikante Effekte der Extrakte von Gentiana, Leontopodium und Centella nachgewiesen:
Zum einen zeigt sich ein signifikanter synergistischer Effekt sowohl 1.) auf die Kollagen-Typ I Expression in der extrazellulären Matrix in humandermalen Fibroblasten, als auch 2.) auf die Hemmung der Freisetzung von Matrix-Melloproteinase 1 (MMP-1), einem wichtigen Abbauenzym von Kollagen 1. Dies ist umso überaschender, da Gentiana, Centella, besonders Edelweiss als Einzelsubstanzen gar entgegengesetzte Wirkungen zeigen (siehe Figur 2), sodass selbst eine positiv-additive Wirkung in einer Kombination nicht zu vermuten war.

Extrinsische Faktoren, allen voran Sonnenlicht, induziert in der Haut Enzyme, die Bestandteile der interzellularen Matrix, etwa Kollagen und Elastin abbauen, u.a. MMP-1.

Auch Zigarettenrauch und weitere Umweltkontaminanten zeigten in verschiedenen epidemiologischen Studien sowie ultrastrukturell eine Induktion verschiedener Matrix-Metalloproteinasen, wie z.B. MMP-1, die somit die Kollagenbiosynthese signifikant reduziert (Thieme, Haut und Alter, Effendy, Isaak et.al. 2005, Physiologie der Hautalterung; Araviiskaia et al. The impact of airborne pollution on skin, JEADV (2019) 10.1111/jdv.15583, https://doi.org/10.1111/jdv.15583).

Folglich sind die Kombinationen der Extrakte von i.) Gentiana und Leontopodium, oder ii.) Gentiana und Centella oder iii.) Gentiana und Leontopodium und Centella besonders geeignet durch die Steigerung der Expression von Kollagen vom Typ I und Inhibition von MMP-1, den Hautalterungsprozess zu verzögern.

Insbesondere in einer geeigneten galenischen Formulierung können sie daher dazu beitragen, die Hautalterung hinauszuzögern (Anti-Aging), und daher ist ebenfalls die Faltenbildung verzögert (Anti-Wrinkle). Ebenfalls ist eine Stresswirkung durch Verschmutzung oder kurzwelliges Licht reduziert, zumindest verzögert (Anti-Pollution, Lichtschutz, insbesondere Blaulicht und IR, UV-Schutz).

Der vorliegenden Erfindung liegt daher die Aufgabe zugrunde, eine kosmetische Zusammensetzung zur topischen Verwendung bereitzustellen, die insbesondere den Hautalterungsprozess verzögern kann.

Die Aufgabe wird durch eine kosmetische Zusammensetzung enthaltend i.) Gentiana und Leontopodium oder ii.) Gentiana und Leontopodium und Centella, bzw. durch die vermittelte technische Lehre mindestens eines Patentanspruches gelöst.

Daher betrifft die Erfindung eine solche kosmetische Zusammensetzung und ein Mittel davon, insbesondere ein Anti-Aging Mittel, ein Anti-Wrinkle Mittel, ein Anti-Pollution Mittel, ein Lichtschutzmittel, insbesondere ein Blaulicht- und IR, UV-Schutzmittel.

In einer bevorzugten Ausführungsform werden die Extrakte aus Gentiana, Leontopodium und Centella mittels eines Auszugsmittels aus dem Pflanzenmaterial bzw. Droge in der Wärme oder Kälte erhalten, welches Wasser und / oder mindestens ein organisches Lösungsmittel enthält, insbesondere C1-C4-Alkohole, vorzugsweise Ethanol. Weiterhin kann Wasser als Auszugsmittel verwendet werden. Die Extrakte können beispielsweise mittels Extraktion, Dekoktion, Perkolation oder Mazeration aus den vorzugsweise getrockneten Pflanzenteilen gewonnen werden. Eine übliche Extraktion ist beispielsweise eine wässrig-ethanolische Extraktion, insbesondere ein Gemisch aus Wasser / Ethanol (50:50, insbesondere im Bereich von 80:20 oder 20:80) z.B. bei 15 bis 80 Grad Celsius und Normaldruck.

Die Extrakte können mit mehrwertigen Alkoholen, beispielsweise Glycerin oder Glykolen und/oder mit Hilfe von antimikrobiellen Hilfsstoffen stabilisiert werden.

Eine bevorzugte Herstellung eines Gentiana Extrakts ist die Dekoktion der oberirdischen Pflanzenteile mit einem Ethanol-Wasser Gemisch. Nach Entfernung des Lösungsmittels wird der Fluidextrakt in Glycerin und/oder Glycolen aufgenommen. Weiterhin ist blauer Enzian bevorzugt, insbesondere die Arten *Gentiana septemfida* und *Gentiana acaulis.*

Eine bevorzugte Herstellung eines Leontopodium Extrakts ist die Dekoktion der oberirdischen Pflanzenteile, insbesondere der Blüten und Blätter, mit einem Ethanol-Wasser Gemisch. Nach Entfernung des Lösungsmittels kann der Fluidextrakt in Glycerin und/oder Glycolen aufgenommen werden. Weiterhin ist die Art *Leontopodium alpinum* bevorzugt.

Eine bevorzugte Herstellung eines Centella Extrakts ist die Mazeration der oberirdischen Pflanzenteile mit einem Ethanol-Wasser Gemisch. Der Extrakt wird filtriert und kann direkt eingesetzt werden. Weiterhin ist die Art *Centella asiatica* bevorzugt.

In einer weiteren bevorzugten Ausführungsform ist das Gewichtsverhältnis in w/w (m/m) der Extrakte aus Gentiana, Leontopodium und Centella, wie folgt: Gentiana : Leontopodium zu 2,5 - 4 : 1 (w/w) oder Gentiana : Centella zu 2,5 - 4 : 1 (w/w) und Gentiana : Leontopodium : Centella zu 2,5 - 4 : 0,5 - 1 : 0,5 - 1 (w/w/w).

Weiterhin ist bevorzugt, dass die Gewichtsmengen in einer kosmetischen Zusammensetzung von Gentiana, Leontopodium und Centella, jeweils mehr als 0,5 Gew. %, vorzugsweise 1 - 2 Gew. %, 1 - 5 Gew. % besonders bevorzugt betragen.

Eine vorteilhafte Rezeptur umfasst neben diesen Wirkstoffen weitere Hilfs- und Zusatzstoffe, als auch Pflegestoffe, wie Öle, insbesondere pflanzliche Samenöle. Eine beispielhafte Zusammensetzung ist in Tabelle 1 (Beispiel 1) angegeben mit Angabe der Funktion der Inhaltsstoffe sowie der eingesetzten Menge in Gew.% bezogen auf die Gesamtmenge.

In einer weiteren Ausführungsform kann die erfindungsgemäße kosmetische oder dermatologische Zusammensetzung zur topischen Verwendung in Form eines Gels, einer Creme oder einer Lotion erfolgen.

Emulgatoren können vorteilhaft gewählt werden aus der Gruppe der nichtionischen, anionischen, kationischen oder amphoteren Emulgatoren.

Erfindungsgemäß bevorzugt sind Emulgatoren, die aus natürlichen Stoffen hergestellt werden, beispielsweise Zuckerester, die basierend auf mit Saccharose veresterten Fettsäuren aus natürlichen Ölen, die in unterschiedlichen Anteilen als Mono-, Di oder auch Triester vorliegen und/oder auf Basis der Aminosäure Glutamin, die beispielsweise mit Kokosölfettsäuren verestert wird. Darüber hinaus können auch andere W/O oder O/W Emulgatoren, z.B. auf Basis von Ethylenoxid, verwendet werden.

Die erfindungsgemäßen kosmetischen Zusammensetzungen können weiterhin kosmetische Hilfsstoffe enthalten, wie sie üblicherweise in solchen Zubereitungen verwendet werden, z. B. Antioxidantien, Vitamine, natürliche und/oder synthetische Duftstoffe, Konservierungsmittel, Farbstoffe, Pigmente, die eine färbende Wirkung haben, Verdickungsmittel, rheologische Additive mineralischen Ursprungs, oberflächenaktive Substanzen, weichmachende, oder andere übliche Bestandteile einer kosmetischen Formulierung wie Alkohole, Polyole, Polymere, Schaumstabilisatoren, Elektrolyte, organische Lösemittel.

In einer weiteren besonderen Ausführungsform besteht die erfindungsgemäße Zusammensetzung vorzugsweise aus natürlich vorkommenden und/oder naturnahen, resp. naturidentischen Inhaltsstoffen.

Nachfolgende Ausführungsbeispiele und Figuren dienen zur Erläuterung der Erfindung, ohne die Erfindung auf diese Beispiele zu beschränken.

Beispiel 1:

**Tabelle 1:**

| Kosmeti sche Zubereitung zur topis chen Anwendung | | | |
|---|---|---|---|
| **no.** | **INCI** | **Function** | **Menge in Gew.%** |
| 1 | Hectorite | Verdicker | 0,5-1,3% |
| 2 | Glyzerin | Feuchthaltemittel | 7,8-12,2% |
| 3 | Aqua | Lösungsmittel | ad 100 |
| 4 | Betaine | Feuchthaltemittel | 0,5-1,3% |
| 5 | Citric Acid | Stabilisator | 0,1-0,4% |
| 6 | Sucrose Polystearate; Cetyl Palmitate | Emulgator | 2,1-3,9% |
| 7 | Cetearyl Alcohol | Emulgator | 2,1-3,9% |
| 8 | Olea Europaea Fruit Oil | hautpflegende Ölkomponente | 6,3-9,5% |
| 9 | Helianthus Annuus Seed Oil | hautpflegende Ölkomponente | 6,3-9,5% |
| 10 | Ricinus Communis Seed Oil | hautpflegende Ölkomponente | 2,1-4,8% |
| 11 | Sodium Stearoyl Glutamate | Emulgator | 0,1-0,4% |
| 12 | Xanthan Gum | Verdicker | 0,5-1,3% |
| 13 | Silica | Verdicker | 0,5-1,3% |
| 14 | Pentylene Glycol | Lösungsmittel | 1,5-4,8% |
| 15 | Glyceryl Caprylate | Emulgator | 0,1-0,4% |
| 16 | Tocopherol | Antioxidans | 0,04-0,07% |
| 17 | Parfum | Duftstoff | 0,3-1,5% |
| 18 | Gentiana septemfida/acaulis Extract | Wirkstoff | 0,5-5% |
| 19 | Centella Asiatica Extract | Wirkstoff | 0,5-2% |
| 20 | Leontopodium Alpinum Extract | Wirkstoff | 0,5-2% |

### Beispiel 1a:

### Kollagen-Typ I Expression in humandermalen Fibroblasten (fixierte Zellen)

### Assay-Bestimmung

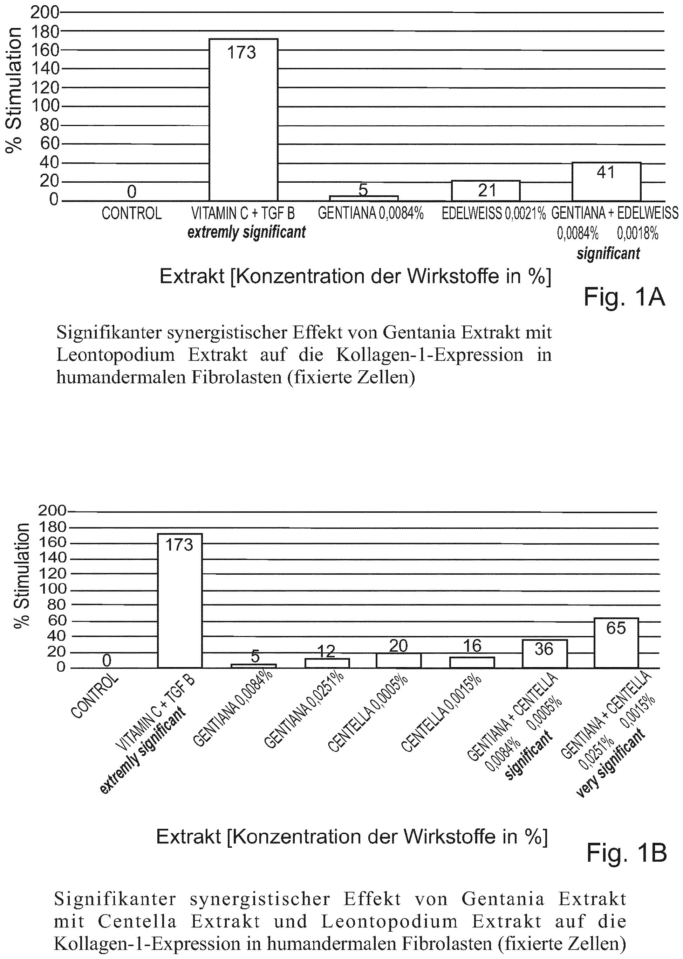

Siehe Figur 1A.

Messung von zwei Extrakten - Enzian und Edelweiss - einzeln und in Kombination.

Positivkontrolle: Vitamin C + TGF β, Negativkontrolle: Assaymedium ohne Wirkstoff, Kultur/Assaymedium: DMEM supplementiert.

Inkubation: 72 h, 96-Mikrotiterplatten.

In situ immunofluoreszenz-labelling:anti-collagen 1/GAR Alexa 488/Hoechst 33258 (bis-benzimide).

Bildanalyse: INCell AnalyzerTM 2200.

Anzahl der Bestimmungen pro Parameter n=3

Einsatzkonzentration der Extrakte:
Einzeln: Enzian 15%iger Extrakt, verdünnt auf 0,0084%
Einzeln: Edelweiss 7%iger Extrakt, verdünnt auf 0,0021%
Kombination: Enzian 15%iger Extrakt, verdünnt auf 0,0084% + Edelweiss 7%iger Extrakt, verdünnt auf 0,00182%

### Beispiel 1b:

### Kollagen-1 Expression in humandermalen Fibroblasten (fixierte Zellen)

### Assay-Bestimmung

Siehe Figur 1B.

Messung von 2 Extrakten - Enzian und Centella - einzeln und in Kombination

Positivkontrolle: Vitamin C + TGF β, Negativkontrolle: Assaymedium ohne Wirkstoff

Kultur/Assaymedium: DMEM supplementiert

Inkubation: 72 h, 96-Mikrotiterplatten

In situ immunofluoreszenz-labelling:anti-collagen 1/GAR Alexa 488/Hoechst 33258 (bis-benzimide)

Bildanalyse: INCell AnalyzerTM 2200

Anzahl der Bestimmungen pro Parameter n=3

Einsatzkonzentration der Extrakte:
Einzeln: Enzian 15%iger Extrakt, verdünnt auf 0,0084%/0,0251%
Einzeln: Centella 9%iger Extrakt, verdünnt auf 0,0005%/0,0015%
Kombination: Enzian 15%iger Extrakt, verdünnt auf 0,0084%/0,0251% + centella 9%iger Extrakt, verdünnt auf 0,0005%/0,0015%

### Beispiel 1c:

### Kollagen-1 Expression in humandermalen Fibroblasten (fixierte Zellen)

### Assay-Bestimmung

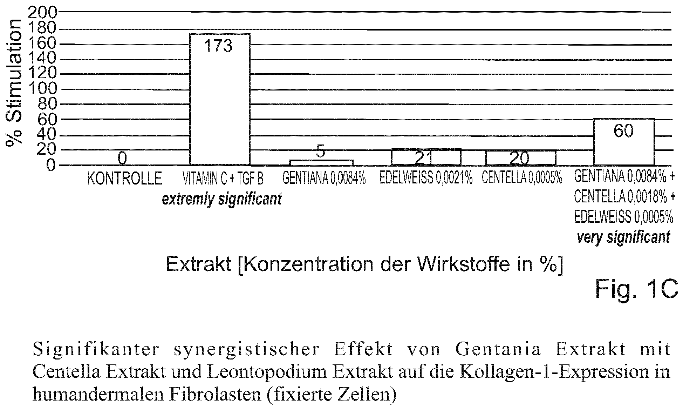

Siehe Figur 1C.

Messung von 3 Extrakten - Enzian, Edelweiss und Centella - einzeln und in Kombination

Positivkontrolle: Vitamin C + TGF β, Negativkontrolle: Assaymedium ohne Wirkstoff

Kultur/Assaymedium: DMEM supplementiert

Inkubation: 72 h, 96-Mikrotiterplatten

In situ immunofluoreszenz-labelling:anti-collagen 1/GAR Alexa 488/Hoechst 33258 (bis-benzimide)

Bildanalyse: INCell AnalyzerTM 2200

Anzahl der Bestimmungen pro Parameter n=3

Einsatzkonzentration der Extrakte:
Einzeln: Enzian 15%iger Extrakt, verdünnt auf 0,0084%
Einzeln: Edelweiss 7%iger Extrakt, verdünnt auf 0,0021%
Einzeln: Centella 9%iger Extrakt, verdünnt auf 0,0005%/0,0015%
Enzian 15%iger Extrakt, verdünnt auf 0,0084% + Edelweiss 7%iger Extrakt, verdünnt auf 0,0018% + Centella 9%iger Extrakt, verdünnt auf 0,0005%

Die Zweier-Kombination der Extrakte Edelweiss + Centella zeigte keinerlei synergistische Effekte auf die Stimulation.

### Beispiel 2:

### Hemmung der Matrix-Metalloproteinase (MMP-1) in gealterten humandermalen Fibroblasten

### Assay-Bestimmung

Siehe Figur 2.

Messung von 3 Extrakten - Enzian, Edelweiss und Centella - einzeln und in 2er und 3er Kombination

Zelltyp: P8-NHDF (als Kontrolle) und P18-F ("gealterte") Fibroblasten

Kultur/Assaymedium: DMEM supplementiert

Kulturbedingungen: 37°C, 5°CO2

Inkubation: 72h, 96-Miikrotiterplatten

Positivkontrolle: Dexamethason 1µM

Negativkontrolle: Assaymedium ohne Verum

MMP-1 release Detektion: ELISA-Kit, Human total MMP-1 DuoSet, R&D Systems, ref. DY901B

Einsatzkonzentration der Extrakte:
Einzeln: Enzian 15%ig, verdünnt auf 0,0084%/0,025%/0,075%e
Einzeln: Edelweiss 7%ig, verdünnt auf 0,0002%, 0,0007%, 0,0021%
Einzeln: Centella asiatica, 9%ige, verdünnt auf 0,0005%, 0,0015%, 0,0045%
Kombination: Enzian 15%ig + Edelweiss 7%ig, verdünnt auf 0,0029%/0,0006% bzw. 0,0084%%0,0018%
Kombination: Enzian 15%ig + Edelweiss 7%ig + Centella 9%ig, verdünnt auf 0,0009%/0,00021&/0,0001% bzw. 0,0029%/0,0006%/0,0002% bzw. 0,0084%/0,0018%/0,0005%

Enzianextrakt allein zeigte in keiner der getesteten Konzentrationen einen signifikanten Effekt auf die MMP-1 Hemmung.

Edelweiss- und Centellaextrakte, jeweils einzeln, zeigten konzentrationsabhängig teilweise signifikante Effekte auf die MMP-1 Hemmung - allerdings sowohl durch Steigerung der Hemmung, aber auch durch im umgekehrten Sinn, d.h. sie verstärkten den Abbau des Kollagen 1.

Die Kombinationen von Enzian- und Edelweissextrakt zeigte signifikante synergistische Effekte auf die MMP-1 Hemmung, während sowohl bei der Kombination von Enzian und Centella und Edelweiss und Centella keine derartigen Effekte nachweisbar waren.

Die Kombinationen von Enzian-, Edelweiss- und Centella Extrakt zeigten in allen getesteten Konzentrationen signifikante bzw. extrem-signifikante Effekte auf die MMP-1 Hemmung, die in den höchsten geteasten Konzentrationen sogar deutlich über dem Effekt des Positivstandards (Dexamethason 1µM) lagen.

Schlussfolgerung:
Diese Daten stützen die Schlussfolgerung, dass die Extrakte von Enzian, Edelweiss und Centella sehr geeignet sind, die Hautalterung zu verzögern. Dabei nehmen sie Einfluss auf das Kollagen 1 im extrazellulären Raum, hier konnte eine Erhöhung der Kollagen 1 Expression nachgewiesen werden.

Darüberhinaus können sie durch Einfluss auf das Enyzm MMP-1, welches u.a. die Spaltung der Peptidbindungen in Proteinen katalysiert, den Abbau des Kollagens durch Hemmung von MMP-1 verzögern.

In einer geeigneten galenischen Formulierung (W/O - O/W Emulsion - Creme, Gel, Lotion - Öl, 2 oder mehrphasiges Produkt, Mizellenwasser, wässriges Produkt mit Lösungsvermittlern) sind sie daher geeignet, die Aufbauprozesse von Kollagen zu unterstützen, die Abbauprozesse desselben zu verzögern und folglich die Hautalterung zu verlangsamen sowie der Entstehung von Fältchen und Falten im Gesicht entgegenzuwirken. Sie sind daher besonders geeignet für "Anti-Aging", "Anti-Wrinkle" und/oder Produkte, die die Hautfestigkeit erhöhen.

## Patentansprüche

1. Kosmetische Zusammensetzung enthaltend i.) Gentiana und Leontopodium, oder ii.) Gentiana und Centella oder iii.) Gentiana und Leontopodium und Centella.

2. Anti-Ageing Mittel aufweisend eine kosmetische Zusammensetzung nach Anspruch 1.

3. Anti-Wrinkle Mittel aufweisend eine kosmetische Zusammensetzung nach Anspruch 1.

4. Anti-Pollution Mittel aufweisend eine kosmetische Zusammensetzung nach Anspruch 1.

5. Lichtschutzmittel, insbesondere Blaulicht- und IR, UV-Schutzmittel aufweisend eine kosmetische Zusammensetzung nach Anspruch 1.

6. Kosmetische Zusammensetzung oder Mittel nach einem der vorstehenden Ansprüche enthaltend
Gentiana : Leontopodium zu 2,5 - 4 : 1 (w/w) oder
Gentiana : Centella zu 2,5 - 4 : 1 (w/w) oder
Gentiana : Leontopodium : Centella zu 2,5 - 4 : 0,5 - 1 : 0,5 - 1 (w/w/w).

7. Kosmetische Zusammensetzung oder Mittel nach einem der vorstehenden Ansprüche enthaltend Gentiana, Leontopodium und Centella, jeweils mehr als 0,5 Gew. %, insbesondere 1 - 2 Gew. % oder 1 - 5 Gew. %.

8. Kosmetische Zusammensetzung oder Mittel nach einem der vorstehenden Ansprüche enthaltend weiter ein Mittel ausgewählt aus der Gruppe Verdicker, Feuchthaltemittel, Lösungsmittel, Stabilisator, Emulgator, hautpflegende Ölkomponente, Antioxidans oder Duftstoff.

9. Kosmetische Zusammensetzung oder Mittel nach einem der vorstehenden Ansprüche enthaltend Hilfs- und Zusatzstoffe.

10. Verwendung von i.) Gentiana und Leontopodium oder ii.) Gentiana und Leontopodium und Centella in einer kosmetischen Zusammensetzung nach einem der Ansprüche 1, 7 bis 9 oder in einem Mittel nach einem der Ansprüche 2 bis 5.
